# FASCICULE DE BREVET EUROPEEN

(11) **EP 1 326 895 B1**
(45) Date de publication et mention de la délivrance du brevet: **14.12.2005**
(21) Numéro de dépôt: 01976422.4
(22) Date de dépôt: 12.10.2001
(51) Int. Cl.: C07K 16/08, A61K 39/42, C12N 5/20, G01N 33/53, C12Q 1/70, A61P 31/20

(54) **ANTICORPS MONOCLONAUX DIRIGES CONTRE DES VIRUS DE L'HEPATITE B**
MONOKLONALE ANTIKÖRPER GEGEN HEPATITIS B VIRUS
MONOCLONAL ANTIBODIES DIRECTED AGAINST HEPATITIS B VIRUSES

(30) Priorité: 20.10.2000 FR 0013454
(43) Date de publication de la demande: 16.07.2003
(73) Titulaire: BIOMERIEUX SA, 69280 Marcy L'Etoile (FR)
(72) Inventeur: LESENECHAL, Mylène, F-69100 Villeurbanne (FR); BATTAIL-POIROT, Nicole, F-69002 Lyon (FR); BECQUART, Laurence, F-69800 Saint Priest (FR)
(74) Mandataire: Grosset-Fournier, Chantal Catherine
(86) Numéro de dépôt international: PCT/FR2001/003169
(87) Numéro de publication internationale: WO 2002/034789

(56) Documents cités:
- WO-A-95/16704
- US-A- 6 030 616
- PAULIJ W P ET AL: "Localization of a unique hepatitis B virus epitope sheds new light on the structure of hepatitis B virus surface antigen." JOURNAL OF GENERAL VIROLOGY, vol. 80, no. 8, août 1999 (1999-08), pages 2121-2126, XP002174119 ISSN: 0022-1317
- COOREMAN MICHEL P ET AL: "Characterization of the reactivity pattern of murine monoclonal antibodies against wild-type hepatitis B surface antigen to G145R and other naturally occurring "a" loop escape mutations." HEPATOLOGY, vol. 30, no. 6, novembre 1999 (1999-11), pages 1287-1292, XP001015475 ISSN: 0270-9139
- JOLIVET-REYNAUD C ET AL: "Localization of hepatitis B surface antigen epitopes present on variants and specifically recognised by anti-hepatitis B surface antigen monoclonal antibodies." JOURNAL OF MEDICAL VIROLOGY, (2001 OCT) 65 (2) 241-9. , XP002195005
- IJAZ SAMREEN ET AL: "Novel immunoassay for the detection of hepatitis B surface 'Escape' mutants and its application in liver transplant recipients." JOURNAL OF MEDICAL VIROLOGY, vol. 63, no. 3, mars 2001 (2001-03), pages 210-216, XP001015468 ISSN: 0146-6615

## Description

Cinq types d'hépatites virales, les hépatites A, B, C, D, E, sont assez bien connues à ce jour. Dans chaque cas, le virus envahit le foie et provoque un état inflammatoire avec une destruction des cellules hépatiques.

L'hépatite B est causée par un virus, le virus de l'hépatite B humain (HBV). Le virus HBV a été découvert par Blumberg et al. A « new » antigen in leukemia sera, JAMA 191 : 541, (1965). Le virus est transmis par voie sanguine, par contact sexuel ou par voie périnatale.

L'infection par HBV dans la majorité des cas n'entraîne aucun symptôme et est responsable d'hépatites aiguës asymptomatiques. L'hépatite aiguë se caractérise par des troubles digestifs, des douleurs abdominales, une coloration des urines et une décoloration des fèces anormales, une asthénie et un ictère. L'hépatite aiguë peut évoluer vers une forme fulminante avec une nécrose rapide du foie.

L'infection virale peut également évoluer vers une forme chronique, soit chez des patients ayant présenté une hépatite aiguë, soit chez des individus pour lesquels l'infection était asymptomatique. Les porteurs chroniques présentent des lésions hépatiques d'importance variable et un risque élevé d'évolution vers une cirrhose et le développement d'un cancer primitif du foie. En Asie et en Afrique où la chronicité des infections est fréquente, les cancers primitifs du foie représentent un problème crucial de santé publique. Par ailleurs, les porteurs chroniques représentent des réservoirs pour le virus et permettent sa propagation transposant le problème de santé publique au niveau mondial.

L'infection par HBV est une des infections virales les plus courantes chez l'homme. C'est une maladie ubiquitaire avec une incidence géographique distincte. En Europe et en Amérique du Nord entre environ 0,1% et 1% de la population est infectée, alors qu'en Asie et en Afrique jusqu'à 20% de la population est porteuse de HBV. On estime à environ 350 millions le nombre de personnes infectées par HBV à travers le monde.

Une hiérarchisation des infections virales suite à une transfusion montre que la transmission de HBV est la première, suivie de HCV et ensuite de HLV.

HBV est un petit virus à ADN de 42 nm de diamètre qui appartient au groupe des virus à ADN hépatoiropes (hepadnavirus) et est classé dans la famille des *Hepadnaviridae*. Sa structure génomique est remarquablement compacte. Le virus comprend une enveloppe externe et une nucléocapside. L'enveloppe est composée principalement de trois antigènes de surface (HBsAgs : hepatitis B surface antigens) qui jouent un rôle majeur dans le diagnostic des infections par HBV. La nucléocapside contient l'antigène du core (HBcAg), une ADN polymérase/transcriptase inverse, ainsi que le génome viral. Le noyau viral constitue l'élément infectieux du virus et la membrane externe porte le principal déterminant antigénique du virus, l'antigène HBs. Le noyau viral demeure à l'intérieur de la nucléocapside. Son diamètre est d'environ 28 nm.

En dépit de sa petite taille (3200 paires de bases) l'ADN circulaire, partiellement double brin, de HBV code pour quatre types de produits viraux à partir de ses gènes chevauchants S, C, P, et X.

Le gène S code pour la protéine d'enveloppe HBsAg exprimée sur la surface externe du virion. La protéine d'enveloppe HBsAg est constituée de deux polypeptides majeurs, un polypeptide de 24 kDa et sa forme glycosylée de 28 kDa. HBsAg contient l'épitope majeur neutralisant de HBV, dénommé déterminant « a », qui correspond aux acides aminés 124 à 147 et est commun à tous les isolats de HBV. Le déterminant « a » est la cible la plus importante pour le diagnostic et la vaccination. Le développement d'anticorps anti-HBs après une infection aiguë ou chronique est généralement associé à un rétablissement et à un pronostic favorable. Les anticorps anti-HBs sont également associés à la production d'anticorps neutralisants après vaccination. La majorité des anticorps anti-HBs trouvés dans le sérum d'individus convalescents ou après vaccination se lient à la région du déterminant « a ». Bien que la structure tridimensionnelle de l'antigène HBs n'ait pas encore définie, des études de structure-fonction indiquent que le déterminant « a » est localisé dans la principale région hydrophile de l'antigène HBsAg (résidus 99 à 169). Il est clair que le déterminant « a » est très structuré parce que la dénaturation de cette zone par alkylation ou réduction donne des particules HBsAg dont l'antigénicité est fortement réduite. Il est probable que des ponts disulfure entre des cystéines sont impliqués dans une conformation correcte. Une structure potentielle du détenninant « a » (résidus 124-147), qui comprend cinq cystéines, impliquerait l'existence de ponts disulfure entre les acides aminés 124 et 137, formant une première boucle, et entre les acides aminés 139 et 147, formant une deuxième boucle. L'intégralité de la séquence du déterminant « a » contribue probablement à la structure antigénique. De plus, l'antigène HBsAg contient soit le déterminant *d* ou *y* qui correspond respectivement à la présence de soit une lysine ou une arginine à la position 122 et, soit le déterminant *w* ou *r* qui correspond respectivement à la présence soit d'une lysine ou d'une arginine à la position 160. Il y a donc quatre sous types antigéniques majeurs de HBsAg, *adw, ayw, adr* et *ayr*, chacun étant associé à une distribution géographique. En amont du gène S, les gènes Pré-S codent pour divers antigènes de surface de HBV.

Le gène P code pour l'ADN polymérase/transcriptase inverse, très importante dans le mécanisme de la réplication virale.

Le gène C code pour deux protéines de la nucléocapside, HBeAg qui est une protéine sécrétée soluble et HBcAg, la protéine core intracellulaire. HBeAg est un marqueur sérologique d'une réplication virale élevée.

Le gène X code pour HBxAg qui a différents effets biologiques et qui peut notamment transactiver la transcription de gènes viraux et cellulaires.

Quand HBV infecte un individu, l'ADN viral se réplique totalement à l'intérieur des cellules hépatiques de l'hôte.

Après infection par HBV, le premier marqueur détectable dans le sérum de patient est l'antigène HBsAg, mais ce marqueur persiste rarement au delà de six mois. Après que l'antigène HBs ait disparu dans le sérum, les anticorps anti-HBsAg deviennent détectables et persistent. Parce que l'antigène HBc est séquestré par l'antigène d'enveloppe HBs, il n'est pas détectable en routine dans les sérums de patients, mais la présence d'anticorps anti-HBc est facilement mise en évidence dans les une à deux semaines suivant l'apparition de l'antigène HBs.

Il est cependant maintenant certain que les tests sérologiques conventionnels, mettant en oeuvre les marqueurs précités, ne permettent pas de détecter les variants de HBV. Or, le fait que des patients porteurs de HBV et ayant développé une hépatite B chronique existent, sans qu'il soit possible de mettre en évidence une infection par HBV à l'aide des marqueurs sérologiques traditionnels, est de la plus haute importance et montre la nécessité de développer de meilleurs tests.

L'existence de variants de HBV est suspectée depuis de nombreuses années. Cette présomption est basée sur la détection d'ADN viral dans le sérum et/ou le foie de patients présentant une hépatite chronique, en l'absence de mise en évidence des marqueurs sérologiques conventionnels (HBsAg et anti-HBc).

L'incapacité à détecter HBsAg chez des patients porteurs de séquences ADN du virus pourrait avoir plusieurs explications, telles qu'une faible expression de l'antigène de surface ou la présence de mutations au niveau du déterminant antigénique de la protéine S. Dans le premier cas, une co-infection virale pourrait supprimer la réplication de HBV (Jilg W et al., J. Hepatol, 1995, 23 : 14-20, Jylberberg et al., Clinical infection diseases, 1996, 23 : 1117- 1125, Ushida et al., J. of Med. Virol. 1997, 52 : 399-405, Hofer et al., Eur. J. Clin ; Microbiol. Infect. Dis., 1998, 17 : 6-13. Une autre explication pourrait être que l'antigène HBs est masqué lors de la formation *in vivo* de complexes immuns avec les anticorps anti-HBs.

Mais, plus récemment, la présence d'HBsAg a été observée dans des sérums anti-HBs de patients. La présence de l'antigène HBsAg chez ces patients pourrait être lié au fait qu'il n'a pas été neutralisé par les anti-HBs présents, suggérant la présence de variants.

La présence de variants ou mutants a été associée à la vaccination et à la thérapie utilisant des anticorps polyclonaux ou monoclonaux, L'analyse de mutants semble montrer qu'ils ont été sélectionnés à partir d'une population mélangée et présentent des mutations ponctuelles causant des substitutions en acides aminés dans le déterminant « a ». Les mutants semblent être le produit de mutations au hasard dans le gène qui conduisent à un pool de génotypes. On pense par ailleurs que la réponse immune est le facteur prédominant dans la sélection des mutants. Généralement, l'addition d'anticorps monoclonaux à des cellules infectées par un virus *in vitro* conduit à une sélection d'isolats qui ne sont pas neutralisés par l'anticorps. Il n'est donc pas surprenant que des anticorps monoclonaux donnés à des patients avec une réplication virale active puissent avoir comme résultat une sélection de mutants d'échappement. Plusieurs mutants d'échappement séparés d'importance clinique ont été trouvés chez des individus vaccinés. Dans plusieurs cas, ils présentaient une mutation ponctuelle au niveau du codon qui code pour l'acide aminé 145 dans le déterminant « a » de l'antigène HBsAg, avec comme résultante le changement d'une glycine en arginine. L'administration de sérum contenant un tel mutant à un chimpanzé a montré que ces agents sont transmissibles. Une autre mutation ponctuelle induisant le remplacement d'une lysine par un acide glutamique à la position 141 du déterminants « a » a également été trouvée chez des sujets vaccinés. Il est donc important de pouvoir détecter de manière sure et fiable, non seulement HBV de type sauvage, mais également les mutants ou variants. En effet, si une mutation significative survient au niveau de l'épitope de HBsAg et qu'elle n'est pas reconnue par les anticorps anti-HBs alors, soit le mutant ne sera pas détecté, soit le test ne sera pas suffisamment sensible. Or, le fait de ne pas détecter un mutant d'échappement a non seulement une incidence pour la personne qui le porte, mais peut également conduire à la transmission de l'infection par les dons de sang, de produits sanguins et d'organes. Par ailleurs, un mutant HBsAg peut infecter des individus même s'ils ont été antérieurement immunisés et présentent une réponse de type anti-HBs. Il a été décrit, dans la demande de brevet internationale WO 94/26904, un anticorps monoclonal qui permet de discriminer la forme HBsAg sauvage d'un mutant d'échappement qui comprend une mutation à la position 122. Mais, il existe un besoin sérieux de caractériser des anticorps monoclonaux qui soient capables de détecter à la fois le type sauvage et les formes mutantes de l'antigène HBsAg pour le développement de tests de diagnostic fiables (Coleman et al. (1999), Journal of Medical Virology 59 : 19-24 ; Ireland et al. (2000), Hepatology 31 : 1176-1181). En effet, dans certains cas, des formes mutantes de HBV affectant la région du gène d'enveloppe codant pour le déterminant « a » ne sont pas reconnues par les anticorps monoclonaux utilisés dans les tests de diagnostic commerciaux (Carman et al. (1990), Lancet 336: 325-329; Seddigh-Tonekaboni et al. (2000), Journal of Medical Virology 60 : 113-121 ; Weinberger et al. (2000), Journal of General Virology 81 : 1165-1174).

Le document US 6 030 616 décrit des anticorps anti-HBsAg capables de se lier à ta forme sauvage de la protéine HBs ainsi qu'à une forme mutante de cette protéine portant la substitution de la glycine en position 145 en arginine.

Le document WO 95/16704 décrit des fragments peptidiques immunogènes de la protéine HBs possédant la propriété d'être des épitopes de lymphocytes T.

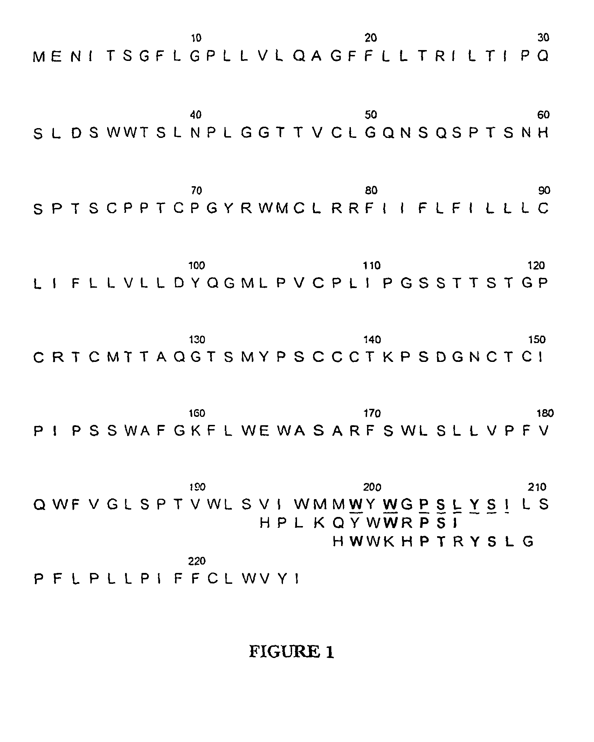

Ainsi la présente invention a pour objet un anticorps monoclonal capable de se lier à un antigène HBsAg de type sauvage et à au moins une, de préférence au moins deux et avantageusement plus de deux, formes mutantes de l'antigène HBsAg, ledit anticorps monoclonal se liant à une séquence peptidique consistant en au moins 6 acides aminés contigus dans la région 199-208 de l'antigène HBsAg, et avantageusement se liant à la séquence peptidique constituée par la région 199-208 de l'antigène HBsAg, selon la numérotation définie dans la figure 1.

Aussi, la présente invention a pour objet un anticorps monoclonal qui est capable de spécifiquement se lier à un antigène HBsAg de type sauvage et à au moins une, de préférence au moins deux et avantageusement plus de deux, formes mutantes de l'antigène HBsAg, ledit anticorps monoclonal se liant spécifiquement à la région 199-208 de l'antigène HBsAg.

La capacité de l'anticorps monoclonal de la présente invention à se lier spécifiquement à des formes sauvage et mutantes de l'antigène HBsAg est reliée au fait qu'il reconnaît une région de l'antigène de surface très conservée, comme montré pour la première fois par les inventeurs par «mapping» d'épitopes, à la fois chez les formes sauvage et mutantes de HBV qui correspond à la région ou épitope localisé entre les acides aminés 199 et 208 de l'antigène HBsAg, et ceci indépendamment de la présence d'autres mutations ou substitutions en acides aminés qui peuvent survenir au niveau du déterminant « a » ou à proximité du déterminant « a » de l'antigène HBsAg. Ainsi, l'anticorps monoclonal de l'invention est capable de reconnaître et de se lier spécifiquement à au moins une forme mutante de l'antigène HBsAg qui comprend au moins une substitution d'un acide aminé dans le déterminant « a » de l'antigène HBsAg et, en particulier, des formes mutantes de l'antigène HBsAg qui présentent au moins une substitution, en acides aminés aux positions 127, 133, 134, 142, 143, 144 et 145 du déterminant « a » de l'antigène HBsAg et éventuellement au moins une autre substitution en acides aminés aux positions 100, 118, 120, 122 de l'antigène HBsAg. Les mutations ou substitutions identifiées sont positionnées par rapport à la séquence en acides aminés de l'antigène HBsAg de type sauvage.

Les formes mutantes qui sont spécifiquement reconnues par Les anticorps monoclonaux de l'invention sont identifiées dans les exemples qui suivent. II s'agit plus précisément des mutants :
- 1043 Sp dans lequel une sérine à la position 143 de l'antigène HBsAg est remplacée par une leucine,
- AP 3.1 dans lequel un acide aspartique à la position 144 de l'antigène HBsAg est remplacée par une alanine,
- Arg 1.2 dans lequel une glycine à la position 145 de l'antigène HBsAg est remplacée par une arginine,
- 1157 Sp dans lequel une proline à la position 127 de l'antigène HBsAg est remplacée par une alanine et une sérine à la position 143 de l'antigène HBsAg est remplacée par une leucine,
- 1056 Sp dans lequel une proline à la position 120 de l'antigène HBsAg est remplacée par une sérine et une sérine à la position 143 de l'antigène HBsAg est remplacée par une leucine, et
- M5 dans lequel une tyrosine à la position 100 de l'antigène HBsAg est remplacée par une sérine, une thréonine à la position 118 de l'antigène HBsAg est remplacée par une valine, une arginine à la position 122 de l'antigène HBsAg est remplacée par une lysine, une méthionine à la position 133 de l'antigène HBsAg est remplacée par une isoleucine, une tyrosine à la position 134 de l'antigène HBsAg est remplacée par une asparagine, une proline à la position 142 de l'antigène HBsAg est remplacée par une sérine, une sérine à la position 145 de l'antigène HBSAg est remplacée par une leucine, et une glycine à la position 145 de l'antigène HBsAg est remplacée par une lysine.

Les mutants précités peuvent être utilisés pour le criblage d'anticorps monoclonaux. Les anticorps de l'invention peuvent être, par exemple, criblés contre de l'antigène HBsAg de type sauvage et contre un ou plusieurs mutants tels que décrits ci-dessus. En particulier, les anticorps de l'invention peuvent être criblés contre de l'antigène HBsAg de type sauvage et contre un ou plusieurs mutants HBsAg chacun ayant une ou plusieurs substitutions à au moins une des positions 127, 133, 134, 142, 143, 144 et 145 de l'antigène HBsAg, plus particulièrement à au moins une des positions 143, 144 et 145 de l'antigène HBsAg et éventuellement au moins une autre substitution à au moins une des positions 100, 118, 120, 122 de l'antigène HBsAg.

En particulier, les anticorps monoclonaux de l'invention sont capables de se lier à un antigène HBsAg de type sauvage et à au moins un mutant HBsAg portant un déterminant « a » codé par des séquences présentant des mutations ponctuelles au niveau d'un ou plusieurs codons codant pour les acides aminés 127, 133, 134, 142, 143,144 et 145 de l'antigène HBsAg. Les anticorps préférentiels de l'invention sont les anticorps référencés 2G2G10 et 8B4H7 qui appartiennent à la classe des immunoglobulines G. L'anticorps monoclonal 2G2G10 appartient à la classe des immunoglobulines IgG2b. L'anticorps monoclonal 8B4H7 appartient à la classe des IgG2a et a été obtenu par traitement aux UV de l'anticorps 2G2G10.

La présente invention englobe également les fragments et dérivés des anticorps monoclonaux de l'invention, en particulier les fragments Fab, Fab', F(ab)2 et scFv (Blazar et al., 1997, Journal of Immunology 159 : 5821-5833 et Bird et al., 1988, Science 242 : 423-426), ainsi que les conjugués. Les dérivés des anticorps monoclonaux de l'invention comprennent, entre autres, les anticorps humanisés. Les méthodes pour produire des fragments d'anticorps monoclonaux et des dérivés d'anticorps monoclonaux, incluant les dérivés humanisés, sont bien connues de l'homme du métier. Les formes « humanisées » d'anticorps non humains, par exemple murins, sont des anticorps chimères qui comprennent une séquence minimale dérivée d'une immunoglobuline non humaine. Pour la plupart, les anticorps humanisés sont des immunoglobulines humaines (anticorps récepteur) dans lesquelles des résidus d'une région hypervariable du récepteur sont remplacés par des résidus d'une région hypervariable d'une espèce donneur (anticorps donneur) non humaine, telle que souris, rat, lapin ou primate non humain, ayant la spécificité, l'affinité et la capacité souhaitées. Dans certains cas, les résidus (FR) de la région Fv de l'immunoglobuline humaine sont remplacés par des résidus correspondants non humains. De plus, les anticorps humanisés peuvent comprendre des résidus qui ne sont pas trouvés dans l'anticorps receveur ou dans l'anticorps donneur. Ces modifications sont effectuées pour améliorer les performances de l'anticorps. En général, l'anticorps humanisé comprendra au moins et de préférence deux domaines variables, dans lesquels tout ou à peu près tout des boucles hypervariables correspondent à une immunoglobuline non humaine et tout ou à peu près tour des régions FR seront celles d'une immunoglobuline humaine. Les anticorps humanisés facultativement pourront aussi comprendre au moins une partie d'une région constante (Fc) d'une immunoglobuline, telle qu'une immunoglobuline humaine (Jones et al., Nature 321 : 522-525 (1986) ; Reichmann et al., Nature 332 : 323-329 (1988) ; et Presta et al., Curr. Op. Struct. Biol. 2 : 593-596 (1992).

Les anticorps monoclonaux de l'invention et, en particulier les anticorps 2G2G10 et 8B4H7, sont produits par des lignées d'hybridomes selon le protocole décrit dans l'exemple 1 qui suit ou selon un protocole dérivé de celui décrit dans l'exemple 1 et l'invention a aussi pour objet un tel procédé de production des anticorps monoclonaux de l'invention qui consiste à produire une lignée d'hybridome en immunisant un animal mammifère, de préférence murin, avec un antigène HBsAg de type sauvage ou une forme mutante d'un antigène HBsAg, éventuellement sous la forme d'un fragment ou d'un dérivé antigénique approprié, en immortalisant les cellules productrices d'anticorps pour former des hybridomes, en criblant les cultures d'hybridomes contre de l'antigène HBsAg de type sauvage et au moins une forme mutante telle que définie précédemment, éventuellement sous la forme d'un fragment ou dérivé antigénique approprié, et en sélectionnant les hybridomes qui produisent des anticorps qui se lient à l'antigène HBsAg de type sauvage et à au moins une forme mutante telle que définie ci-dessus.

L'invention englobe également un hybridome capable de produire un anticorps monoclonal de l'invention et un procédé pour produire un anticorps monoclonal de l'invention, qui consiste à cultiver un hybridome ou susceptible d'être obtenu selon un procédé tel que défini ci-dessus *in vitro* ou *in vivo* et à récupérer l'anticorps monoclonal à partir du milieu de culture ; ainsi que l'anticorps monoclonal susceptible d'être obtenu par le procédé de culture d'un hybridome.

Les anticorps monoclonaux de l'invention sont utilisables dans un immunoessai pour la détection et/ou quantification de l'antigène HBsAg dans un échantillon présumé le contenir et donc pour la détection de virus HBV (sauvage et/ou mutants). De tels essais sont applicables au diagnostic clinique et au criblage de produits sanguins. Aussi, la présente invention concerne un procédé de détection et/ou de quantification d'antigène HBsAg dans un échantillon, qui consiste à mettre en contact l'échantillon avec au moins un anticorps monoclonal de l'invention, ou un fragment ou dérivé d'un tel anticorps monoclonal et à mettre en évidence la présence d'au moins un complexe antigène/anticorps, fragment ou dérivé.

Dans un mode de réalisation du procédé de l'invention, l'échantillon est mis en contact avec un anticorps monoclonal de l'invention, ou un fragment ou dérivé de celui-ci et avec au moins un autre anticorps choisi parmi les anticorps polyclonaux ou monoclonaux anti-HBsAg dirigés contre une région ou épitope différent de celui reconnu par les anticorps monoclonaux, fragments ou dérivés de l'invention.

De tels procédés sont réalisés selon la technique dite sandwich ou de compétition bien connues de l'homme du métier, en une ou deux étapes, en phase homogène ou hétérogène. De plus, les procédés de l'invention peuvent être couplés, dans un même essai, à la détection d'anticorps anti-HBc dans l'échantillon à analyser.

Les compositions diagnostiques correspondantes comprennent au moins un anticorps monoclonal de l'invention, ou un fragment ou dérivé de celui-ci, éventuellement en association avec au moins un réactif pour la détection d'anticorps anti-HBc et peuvent comprendre de plus au moins un autre anticorps monoclonal ou polyclonal pour la détection de l'antigène HBsAg qui reconnaît une région ou épitope différent de celui reconnu par les anticorps monoclonaux, fragments ou dérivés de l'invention. Les anticorps, fragments ou dérivés de l'invention peuvent être immobilisés sur une phase solide, comme anticorps de capture de l'antigène HBsAg ou utilisés en détection quand marqués avec tout marqueur approprié. Il est également envisagé d'utiliser les anticorps, fragments ou dérivés de l'invention comme phase de capture de l'antigène HBsAg et d'utiliser au moins un autre anticorps monoclonal ou polyclonal dirigé contre un épitope différent de celui reconnu par les anticorps de l'invention comme conjugué de détection, ou inversement. De même, les anticorps, fragments ou dérivés de l'invention et au moins un autre anticorps monoclonal ou polyclonal dirigé contre un épitope différent de celui reconnu par les anticorps de l'invention peuvent être immobilisés sur une même phase solide. Et de plus, un réactif capable de capturer les anticorps anti-HBc dans l'échantillon peut être immobilisé sur cette même phase solide ou être inclus dans une composition de l'invention mais en étant immobilisé sur une phase solide différente de celle sur laquelle sont immobilisés les anticorps, fragments ou dérivés de l'invention et éventuellement au moins un autre anticorps monolonal ou polyclonal anti-HBsAg reconnaissant un épitope différent de celui reconnu par les anticorps de l'invention.

L'invention concerne aussi un antisérum approprié pour une utilisation thérapeutique ou prophylactique pour une immunisation passive contre HBV qui comprend un anticorps monoclonal de l'invention ou un fragment ou dérivé d'un tel anticorps ou leurs combinaisons. L'antisérum peut de plus comprendre au moins anticorps monoclonal ou un anticorps polyclonal ou un mélange d'anticorps monoclonaux et polyclonaux anti-HBsAg, autres que l'anticorps monoclonal, fragment ou dérivé de l'invention.

Une composition pharmaceutique appropriée pour une utilisation thérapeutique ou prophylactique pour une immunisation passive contre HBV comprend un anticorps monoclonal, ou un fragment ou dérivé de l'invention ou leurs combinaisons, en mélange avec un véhicule pharmaceutiquement acceptable.

Par « véhicule pharmaceutiquement acceptable » on entend les supports et véhicules administrables à l'être humain ou à un animal, tels que décrits par exemple dans Remington's Pharmaceutical Sciences 16^{th} ed., Mack Publishing Co.

L'invention permet donc de plus une méthode d'immunisation passive à but thérapeutique ou prophylactique conter HBV qui consiste à administrer à un individu une quantité thérapeutiquement ou prophylactiquement efficace d'un anticorps monoclonal, fragment ou dérivé de l'invention ou leurs combinaisons. Un ou plusieurs autres anticorps monoclonaux ou polyclonaux anti-HBSAg et dirigés contre un épitope différent de celui reconnu par les anticorps, fragments ou dérivés de l'invention peuvent également être administrés.

L'invention inclut également un anticorps anti-idiotype ou anti-idiotypique à un anticorps de l'invention tel que défini ci-dessus. L'anticorps anti-idiotype est un anticorps produit contre un autre anticorps et qui réagit avec le site spécifique de liaison antigénique de ce dernier ou idiotype. Les anticorps anti-idiotype fonctionnent comme l'antigène reconnu par l'idiotype. De tels anticorps sont particulier utiles dans le cas d'épitopes conformationnels. Les anticorps anti-idiotype qui représentent l'image interne de pathogènes externes, tels que les virus, sont utilisés comme « substituts » des antigènes pour la vaccination. L'importance des anticorps anti-idiotypes *in vivo* a été démontrée dans de nombreuses expérimentations. L'administration d'anticorps anti-idiotype *in vivo* a pour effet soit une suppression ou une augmentation des réponses à l'idiotype spécifique. Les anticorps anti-idiotypes de l'invention sont capables d'induire chez l'animal une réponse par production d'anticorps anti-anti-idiotypes qui reconnaissent l'épitope 199-208 de l'antigène HBsAg et à ce titre ils sont capables d'immuniser un animal contre HBV, voire de neutraliser une infection par HBV. Aussi, la présente invention a pour objet une composition vaccinale qui comprend une quantité suffisante, pour immuniser un animal contre HBV, d'une composition active comprenant un anticorps monoclonal anti-idiotype qui induit chez l'animal la production d'un anticorps anti-anti-idiotype qui reconnaît la région 199-208 de l'antigène HBsAg ou un fragment Fab dudit anticorps anti-idiotype ou leur mélange et un adjuvant pharmaceutiquement acceptable, par exemple de l'hydroxyde d'aluminium ou du phosphate d'aluminium. Les adjuvants pharmaceutiquement acceptables sont bien connus de l'homme du métier et on peut donner à titre de référence les adjuvants cités dans Remington's Pharmaceutical Sciences 16^{th} ed., Mack Publishing Co. Si désiré, le vaccin peut contenir des quantités mineures de substances auxiliaires comme des agents mouillants ou émulsifiants, des agents qui tamponnent le pH. Le procédé pour immuniser un animal contre HBV consiste à lui administrer, en une ou plusieurs administrations, une composition vaccinale telle que définie ci-dessus. Bien entendu, après les essais *in vivo* sur les animaux, le but ultime d'une vaccination contre le virus HBV est de prévenir une infection chez l'homme. Aussi, le terme « animal » tel qu'utilisé ci-dessus fait référence à la fois aux animaux et à l'être humain. Le vaccin est préparé en mélangeant l'anticorps anti-idiotype de l'invention ou son fragment Fab dans un diluent approprié, qui peut être un tampon phosphate aqueux ou autre, de façon à obtenir une concentration finale de l'ordre de 1 mg par ml de protéine. Pour une administration a un homme de taille normale, par exemple, 50 µl (50 µg) à 100 µl (100 µg) de la solution sont dilués dans 4 500 µl et mélangés avec 500 µl d'un adjuvant pharmaceutiquement aceptable (pH 6,0 + 0,1). La dose variera en fonction de l'animal, de son âge et de son poids.

Les anticorps monoclonaux idiotype de l'invention sont produits par des techniques bien connues de l'homme du métier. L'épitope ou région 199-208 de l'antigène HBsAg est obtenu et purifié par toute technique actuellement disponible. L'anticorps idiotype (Ab1) est produit en immunisant un animal, de préférence murin, et en particulier une souris. avec l'épitope précité, comme antigène. Les cellules de rate de l'animal sont ensuite identifiées, isolées et fusionnées avec des cellules de lymphome ou de myélome en présence d'un agent de fusion tel que le polyéthylène glycol (Köhler et Milstein, Nature 256:459 (1975)). Les cellules fusionnées sont ensuite incubées dans un milieu sélectif qui inhibe la croissance des cellules malignes non fusionnées. Les cellules d'hybridome sont clonées par dilution limitante et testées pour la sécrétion d'un anticorps monoclonal de spécificité recherchée.. Les anticorps monoclonaux peuvent également être obtenus par croissance d'ascite *in vivo*. L'anticorps idiotype Ab1 est utilisé pour produire un anticorps anti-idiotype (Ab2) qui possède les propriétés d'immunisation, voire de neutralisation, contre HBV. Les anticorps anti-idiotype Ab2 sont obtenus par les mêmes procédés que ceux utilisés pour la production des anticorps Ab1, mais l'anticorps Ab1 est alors l'immunogène utilisé.

L'invention a également pour objet l'épitope conservé de l'antigène HBsAg mis en évidence par les inventeurs dont la séquence peptidique, représentée dans l'identificateur de séquences SEQ ID NO : 1, commence à l'acide aminé 199 et se termine à l'acide aminé 208 de l'antigène HBsAg et son utilisation comme peptide immunogène, ainsi que des peptides immunogènes caractérisés en ce que leur séquence peptidique consiste en l'épitope référencé en SEQ ID NO : 1, ou les peptides immunogènes illustrés en SEQ ID NO : 2 et SEQ ID NO : 3. Dans la comparaison des séquences SEQ ID NO : 1 et SEQ ID NO : 2 illustrée à la figure annexée, la séquence SEQ ID NO : 2 comporte trois acides aminés, W (tryptophane), P (proline), S (sérine) qui sont strictement homologues ou identiques aux acides aminés correspondants de SEQ ID NO :1 et un acide aminé 1 (isoleucine) qui est équivalent à L (leucine) de SEQ ID NO :1. Dans la comparaison des séquences SEQ ID NO :1 et SEQ ID NO : 3 illustrée à la figure annexée, la séquence SEQ ID NO : 3 comporte quatre acides aminés qui sont strictement homologues ou identiques à cew de SEQ ID NO : 1, il s'agit des acides aminés W (tryptophane), P (proline), Y (tyrosine), S (sérine), et deux acides aminés T (thréonine) et L (leucine) qui sont équivalents respectivement à S (sérine) et I (isoleucine) de SEQ ID NO : 1. Les acides aminés dits équivalents sont des acides aminés qui peuvent se substituer les uns aux autres sans affecter le pouvoir immunogène des peptides. De tels peptides immunogènes sont utilisés pour la production d'anticorps monoclonaux, de fragments d'anticorps monoclonaux ou de dérivés d'anticorps monoclonaux répondant aux définitions données précédemment et capables de reconnaître à la fois la forme sauvage et au moins une forme mutante de l'antigène HBsAg. La production d'anticorps monoclonaux, de fragments ou dérivés d'anticorps monoclonaux est bien connue de l'homme du métier. On peut citer à titre d'exemple Köhler et Milstein, Nature 256 : 45-47 (1975) et European Journal of Immunology 6 : 511-519 (1976) ainsi que Galfre G. et al. Nature, 266: 522-550 (1977). L'épitope peut être produit par synthèse chimique ou par recombinaison génétique ou par une combinaison de différentes méthodes. La séquence codant pour l'épitope de l'invention peut ainsi être exprimée dans une cellule hôte appropriée sous le contrôle d'un promoteur approprié. Les cellules hôtes comprennent tout organisme unicellulaire capable de transcrire et de traduire des molécules d'ADN recombinant, telles que les cellules issues d'un organisme procaryote ou eucaryote incluant les cellules de mammifères, de levures et de bactéries, et permettant l'expression du fragment du gène S codant pour l'épitope de l'invention, le fragment du gène étant placé sous le contrôle des éléments nécessaires à son expression. Par ailleurs, les peptides immunogènes SEQ ID NO : 2 et SEQ ID NO : 3 peuvent aisément être obtenus par synthèse chimique.

L'invention concerne enfin une sonde nucléique ou amorce, capable de s'hybrider, dans des conditions de stringence déterminées, notamment stringentes, à une séquence nucléotidique ADN qui code pour l'épitope représenté en SEQ ID NO : lou à la séquence nucléotidique ADN complémentaire de ladite séquence nucléotidique codant pour l'épitope représenté en SEQ ID NO : 1 ou au produit de transcription ARN desdites séquences nucléotidiques ADN.

L'invention concerne également une sonde nucléique ou amorce capable de s'hybrider, dans des conditions de stringence déterminées, notamment stringentes à une séquence nucleotidique ADN représentée en SEQ ID NO : 4 qui code pour l'épitope représenté en SEQ ID NO : 1 ou à la séquence nucléotidique ADN complémentaire de SEQ ID NO: 4 ou au produit de transcription ARN desdites séquences nucléotidiques ADN ; une composition diagnostique comprenant au moins une sonde ou au moins une amorce telle que définie ci-dessus pour la détection et/ou la quantification de virus HBV (sauvage et/ou mutants) dans un échantillon biologique et un procédé de diagnostic d'ADN et/ou d'ARN de virus HBV (sauvage et/ou mutants) dans un échantillon biologique, selon lequel on prélève un échantillon biologique, tel que sérum, plasma ou échantillon tissulaire chez un patient suspecté d'être infecté par au moins un virus HBV, on traite si nécessaire ledit échantillon pour en extraire l'ADN et/ou l'ARN, on met en contact ledit échantillon avec au moins une sonde ou au moins une amorce, dans des conditions de stringence déterminées et on détecte et/ou quantifie l'ADN et/ou ARN dans l'échantillon, soit par mise en évidence d'une hybridation dudit ADN et/ou ARN viral avec au moins une sonde, soit par amplification dudit ADN et/ou ARN. Dans le cas de l'utilisation de sondes nucléotidiques, la mise en évidence de la formation du complexe d'hybridation peut être effectuée directement par utilisation d'une sonde complémentaire ou sensiblement complémentaire de la séquence de la cible et marquée par tout marqueur approprié ou encore par mise en oeuvre de la technique dite « sandwich » en une ou deux étapes qui consiste à utiliser une sonde de capture complémentaire ou sensiblement complémentaire d'une partie de la séquence de la cible et une sonde marquée par tout marqueur approprié ou sonde de détection complémentaire ou sensiblement complémentaire d'une autre partie de la séquence de la cible. Dans le cas d'utilisation d'amorces, celles-ci peuvent être directement marquées pour la mise en évidence d'un produit d'amplification marqué ou ne pas être marquées, auquel cas les produits d'amplification générés peuvent être sélectionnés en fonction de la taille attendue par passage sur gel d'acrylamide ou analysés à l'aide de sondes de détection appropriées.

A partir de la sélection de la séquence nucléotidique ADN qui code pour l'épitope 199-208 de l'invention, correspondant aux nucléotides 751-780 du génome de HBV décrit par Galibert et al. (Nature 280 : 646-650 (1979), représentée en SEQ ID NO : 4, de sa séquence complémentaire et de la séquence ARN correspondante, il est à la portée de l'homme du métier de déterminer et obtenir des sondes nucléotidiques ou des amorces capables de s'hybrider à SEQ ID NO : 4, à sa séquence complémentaire ou à la séquence ARN correspondante. On peut citer à titre d'exemples pour la production d'oligonucléotides l'ulilisation d'enzymes de restriction et la synthèse chimique sur synthétiseur automatique. Les sondes et amorces susceptibles de s'hybrider dans des conditions de stringence déterminées à une séquence nucléotidique d'ADN ou d'ARN de l'invention font partie de cette définition. Il est à la portée de l'homme du métier de définir les conditions de stringence appropriées. Des conditions de stringence caractéristiques sont celles qui correspondent à une combinaison de la température et de la concentration saline choisie approximativement entre 12 à 20°C sous le Tm (« melting température ») de l'hybride à l'étude. Tout ceci fait partie des connaissances générales de l'homme du métier et on peut citer à titre d'illustralion l'enseignement donné dans le livre intitulé « DNA PROBES » de George H. Keller et Mark M. Manak, Second Edition, édité en 1993 par Stockton Press, 49 West 24^{th} St., New York, N.Y. 10010, USA (voir plus précisément: Section l-Molecular Hybridization Technology, pp. 1-21).

### Figure 1

La figure 1 annexée représente la séquence en acides aminés de l'antigène HBsAg. Sur cette figure est localisé l'épitope 199-208, mis en évidence par cartographie ou « mapping » d'épitopes comme illustré dans les exemples qui suivent. Les deux clones HWWKHPTRYSLG et HPLKQYWWRPSI qui présentent des acides aminés communs ou homologues ou identiques et des acides aminés équivalents aux acides aminés de la séquence de l'antigène HBsAg de type sauvage dans la région comprise entre les acides aminés 194-209 de la séquence en acides aminés de l'antigène HBsAg sont représentés en dessous de la séquence de l'antigène HBs Ag de type sauvage. Les acides aminés communs ou homologues ou identiques et équivalents sont représentés en caractère gras dans la figure 1.

### Exemples.

### Exemple 1 : Production et caractérisation des anticorps monoclonaux.

Des souris femelles BALB/c JYco, âgées de 4 à 6 semaines (IFFA Credo), ont été immunisées par injection intrapéritonéale de 50 µg d'HBsAg *ay* recombinant émulsifié avec un volume égal de l'adjuvant complet de Freund. La protéine recombinante HBsAg *ay* a été obtenue auprès de Pasteur Mérieux Connaught. Cette protéine correspond à la souche *ayw* séquencée par Gallibert et al. (Nature 280 : 646-650 (1979)) et a été exprimée dans des cellules CHO (Michel et al., PNAS 81/7708-7712 (1984)). Elle est composée des régions pré-S et S. Les 55 acides aminés situés en position NH₂ terminale correspondent à la région pré-S2.

Trois injections ont ensuite été effectuées toutes les deux semaines en présence d'adjuvant incomplet. Quatre jours après la dernière injection, les cellules de la rate ont été prélevées et fusionnées avec la lignée cellulaire de myélomes de souris Sp 2/0-Ag14, selon la technique décrite par Köhler & Milstein (Nature 256 : 45-47 (1975) ; European Journal of Immunology 6 : 511-519 (1976). Après avoir cultivé les cellules pendant 12 à 14 jours, les surnageants de culture ont été criblés par un test ELISA indirect avec l'antigène HBsAg plasmatique *ay* recombinant fixé sur la phase solide. Mille cinquante huit surnageants de culture, dilués au 1/10^{éme}, ont ainsi été criblés. Soixante six clones positifs ont été sélectionnés pour lesquels la DO à 405 nm était d'environ 2, soit six fois supérieure à la valeur seuil, à cette même longueur d'onde, de 0,3. Les soixante six surnageants de culture des clones positifs, dilués au 1/10^{éme}, ont ensuite été criblés par un test ELISA indirect avec de l'antigène HBsAg plasmatique *ay* natif. Vingt clones ont été sélectionnés pour lesquels la DO à 405 nm était supérieure à 2, soit 24 fois supérieure à la valeur seuil, à cette même longueur d'onde, de 0,1. Les surnageants de culture de 12 sur ces 20 clones, dilués au 1/10^{éme}, ont ensuite été testés par un test ELISA indirect, respectivement avec de l'antigène HBsAg *ay* natif et de l'antigène HBsAg *ad* natif, fixé sur la phase solide. Six clones ont été sélectionnés dans chacun de ces tests, pour lesquels la DO à 405 nm était de 1,2 ou plus pour l'essai avec l'antigène HBsAg ay natif et d'environ 1,1 pour l'essai avec l'antigène HBsAg *ad* natif, à une dilution au 1/1600^{éme} du surnageant de culture, soit une DO environ dix fois supérieure à la valeur seuil de 0,1. Quatre de ces six clones positifs ont été sous-clonés deux fois par dilution limitante et produits sous forme d'ascite. Les ascites ont été obtenus à partir de souris, ayant reçu au préalable une injection de Pristane (nom commercial), et auxquelles 10⁶ cellules d'hybridomes ont été injectées.

Les quatre IgGs ainsi obtenues ont été purifiées sur une colonne de sépharose couplée à la protéine A 4FF, selon le protocole fourni par le fabriquant (Pharmacia) et ont été utilisées pour la suite des expériences. En final, l'anticorps monoclonal 2G2G10, qui appartient à la classe des immunoglobulines IgG2b, a été retenu en raison de ses excellentes performances.

### Exemple 2 :Changement de classe de l'anticorps 2G2G10.

La classe de l'anticorps monoclonal 2G2G10 a été changée d'IgG2b en IgG2a par traitement aux UV selon le protocole de Rosén et Klein (Nature 306 : 189-190 (1983)). L'anticorps modifié a été nommé 8B4H7.

### Exemple 3 : Criblage d'une banque de peptides portés par des phages.

La banque Ph.D.-12™ commercialisée par New England Biolabs Inc. a été criblée par les anticorps monoclonaux 2G2G10 et 8B4H7 biotinylés selon la méthode décrite par Gretch et al. (Analytical Biochemistry 163: 270-277 (1987)). C'est une banque combinatoire de dodecapeptides exprimés en N-terminal d'une protéine mineure d'enveloppe (pUI) du phage M13. Elle contient environ 4.10¹² phages/ml, représentant 1,9.10⁹ séquences différentes.

Lors du premier « biopanning », 10 µg de streptavidine en tampon NaHCO₃ 0,1 M (pH 8,6) sont fixés dans une boîte de Pétri de 35 mm de diamètre, pendant une nuit, à 4°C, en chambre humide, sous agitation. La boite est ensuite saturée pendant 2h à 4°C avec une solution de saturation [5mg/ml BSA; 0,02% NaN₃ dans NaHCO₃ 0,1M (pH 8,6)]. Après 6 lavages en TBS (Tris Buffer Saline) [Tris 0,5 M; NaCl 0,75 M, pH 7,5]-Tween 0,1%, les anticorps monoclonaux biotinylés 2G2G10ou 8B4H7 sont incubés à une concentration de 170 nM en TBS Tween 0,1% avec 0,02% NaN₃ et 1 mg/ml de BSA pendant 1 nuit à 4°C, en chambre humide et sous agitation. Pour bloquer les sites non occupés par l'anticorps, 40 µg de bioline sont ajoutés et incubés pendant 1 heure à 4°C, en chambre humide et sous agitation. Après 6 lavages en TBS Tween 0.1%, 10 µl de la banque (4.10⁹ phages) dilués dans du TBS Tween 0.1 %-biotine 0,1 nM sont incubés 1 heure à température ambiante, sous agitation. Les phages non fixés sont ensuite éliminés par 10 lavages en TBS Tween 0,1%. L'élution des phages fixés se fait par une incubation de 7 minutes avec le tampon d'élution [0,1M HCl-Glycine; pH 2,2; 1 mg/ml BSA; 0.1 mg/ml rouge de phénol]. L'éluat est neutralisé par 200 µl de Tris-HCl 1M pH 9,1.

Les éluats correspondant aux criblages de la banque, soit avec 2G2G10, soit avec SB4H7 sont amplifiés séparément par infection de la souche ER2537 d'*Escherichia coli* en milieu LB (tryptone 10 g/l ; extrait de levure 5 g/l; NaCl 10 g/l; pH 7,5]. Après 4h30 d'incubation à 37°C à 250 tpm, les débris cellulaires sont éliminés par deux centrifugations successives de 10 min. à 10 000 tpm. Les phages contenus dans les surnageants sont précipités deux fois avec 1/6^{ème} de Polyéthylène glycol/NaCl [Polyéthylène glycol 16,7 %, Nacl 3,3 M]. Après une centrifugation de 15 min. à 14 000 tpm, les culots sont repris par 200 µl de TBS-NaN₃ 0,02%. 100 µl de ces éluats amplifiés sont utilisés pour les deuxièmes biopannings.

Pour les 2^{ème}, 3^{ème} et 4^{ème} « biopannings », 100 µl des éluats amplifiés précédents sont préincubés avec respectivement 100 nM, 1 nM et 0,1 nM des anticorps biotinylés 2G2G10 ou SB4H7 pendant une nuit à 4°C, pendant que 10 µg de streptavidine sont fixés sur une boîte de Pétri, comme précédemment. Les mélanges phages/anticorps sont ajoutés après saturation des boîtes, pendant 15 minutes à température ambiante. La suite du protocole est semblable à celui des premiers « biopannings ».

### Exemple 4 : Clonage et amplification des clones de phages.

Une culture d'*E. coli* est incubée à 37°C à 250 tpm jusqu'à ce que la densité optique (DO) atteigne 0.5. Pendant la croissance bactérienne, de l'agarose top [11 LB ; 10 g bacto-tryptone ; 5 g extrait de levure ; 5 g NaCl ; 1 g MgCl₂, 6H₂O ; 7g agarose] est fondu et aliquoté par 3 ml, et conservé à 55°C. Lorsque les cellules sont prêtes, 200 µl de culture sont incubées de 1 à 5 minutes à température ambiante avec 10 µl des éluat obtenus après les 4^{ème} biopannings et dilués en milieu LB. Le mélanges sont ensuite transférés dans 2 tubes d'agarose, vortexés et rapidement étalés sur des boîtes LB IPTG/XGal. Les boîtes sont incubées une nuit à 37°C.

36 colonies choisies au hasard sur les boîtes correspondants au criblage par 2G2G 10 ou SB4H7 sont amplifiées par infection d'1 ml d'une culture d'.*E. coli* pendant 4 à 5 heures à 37°C, puis les cellules sont éliminées par centrifugation 30 s à 14 000 tpm. 500 µl du surnageant sont utilisés pour le séquençage, le reste est centrifugé de nouveau, dilué 1:1 dans du glycérol et conservé à -20°C.

2 µl de phages conservés en LB/Glycérol sont amplifiés par infection de 1,7 ml d'une culture de *E. coli* pendant 24 heures à 37°C et 250 tpm. Les cellules sont éliminées par centrifugation min. à 14 000. tpm. Le surnageant est précipité par du PEG/NaCl. Après une centrifugation de 15 min. à 14 000 tpm, le culot est repris par 500 µl de TBS. Ces solutions de phages sont dosées par spectrométrie d'absorption à 269 nm et utilisées pour les tests en ELISA.

### Exemple 5 :Séquençage des séquences insérées dans les clones phagiques.

L'extraction et la purification de l'ADN des différents clones sont effectuées à partir de 500 µl de surnageant qui sont précipités par 200 µl de PEG/NaCl pendant 10 min. à température ambiante. Après centrifugation 10 min. à 14 000 tpm, le culot est repris par 100 µl de Tris-HCl 10 mM pH 8,0, EDTA 1 mM, NaI 4M et 250 µl d'éthanol absolu puis incubé 10 min. à température ambiante. Après centrifugation 10 min. à 14 000 tpm, le culot est lavé une fois par 500 µl d'éthanol 70%, puis séché 10 min. sous vide. 11 est finalement repris par 30 µl de Tris-HCl 10 mM pH S,0, EDTA 1 mM.

Le séquençage nucléoticiique de l'insert est effectué selon la méthode modifiée de Sanger avec un séquenceur automatique (modèle 377A, Perkin Elmer) en utilisant le kit de séquençage "Big Dye™ Terminator Cycle Sequencing Ready Reaction" (Perkin-Elmer) et l'amorce 5'HO-CCCTCATAGTTAGCGTAACG-OH3' correspondant à la séquence sauvage du phage. Le mélange réactionnel est composé de 5 µl d'ADN à séquencer, 2,5 pmoles d'oligonucléotides dans 2,5 µl d'eau, 8 µl de mélange "Big Dye™" contenant les didésoxyribonucléotides et la Taq™ polymérase, et 4.5 µl de H₂O. Les 25 cycles de séquençage (30 s à 96°C, 15 s à 50°C, 4 min. à 60°C) sont effectués dans un appareil PCR (Polymerase Chain Reaction) Trioblock de Biométra. Les fractions de séquence sont purifiées sur colonne Sephadex G50 équilibrée avec du tampon TE [tris-Hcl 10mM pH8, EDTA 1 mM], séchées et conservées à -20°C juqu'au séquençage. Les séquences protéiques sont déduites des séquences nucléotidiques avec le logiciel Geneworks,

### Exemple 6 : Analyse immunologique des clones de phages.

100µl des anticorps anti-HBsAg 2G2G10 ou 8B4H7 ou d'un anticorps non pertinent utilisé comme contrôle négatif à la concentration finale de 100 µg/ml en tampon NaH.CO₃ 0,1 M sont fixés pendant une nuit à 4°C, en chambre humide, sur des rangées de puits de plaques ELISA "Nunc Maxisorb". La plaque est ensuite saturée 2 heures à 4°C en solution de saturation [NaHCO₃ 0,1 M ; BSA 50 mg/ml ; NaN₃ 0,02 %]. Après 4 lavages en TBS-Tween 0.5 %, 100 µl de différentes concentrations de phages dilués en TBS Tween (1.10¹², 2,5.10¹¹, 6,2.10¹⁰ et 4.10⁹ phages/ml) sont ajoutés par puits et incubés 2 heures à température ambiante, sous agitation. Après 4 lavages en TBS Tween 0.5 %, 100 µl/puits d'anticorps anti-M13 couplé à la peroxydase et dilué au 1/5000 en solution de saturation sont incubés pendant 2 heures à température ambiante, sous agitation. Après incubation de 1 heure à 37°C, la plaque est lavée 4 fois en TBS-Tween 0,5 %. Pour la révélation, la plaque est incubée avec une solution d'o-phénylènediamine et de peroxyde d'hydrogène (kit colorEIA. bioMérieux) pendant 10 min. à l'obscurité. La réaction est stoppée par de l'acide sulfurique 1,8 N et la plaque est lue à 492 nm, avec un lecteur de plaques ELISA (Axia microreader). Pour chaque dilution de chaque clone les résultats sont exprimés par la différence des valeurs obtenues entre l'anticorps anti-HBsAg et l'anticorps contrôle. Les résultats sont ensuite confirmes en testant les dilutions optimales en triplicate.

Aucun clone immunoréactif n'a pu être sélectionné par l'anticorps 2G2G10. Par contre, l'anticorps équivalent SB4H7 ayant le même paratope mais de classe IgG 2a a permis de sélectionner des clones immunoréactifs (tableau 1). De plus deux de ces clones (HWWKHPTRYSLG et HPLKQYWWRPSI) sont aussi reconnus par l'anticorps 2G2G10.

**Tableau 1**

| Fréquence et immunoréactivité des clones phagiques sélectionnés avec l'anticorps 2G2G 10 et l'anticorps modifié 8B4H7. | | | | |
|---|---|---|---|---|
| Séquence des clones | Fréquence des clones | Réactivité avec 8B4H7^{a} | | Réactivité avec 2G2G10^{b} |
| HKMHSHPRLTSP | 13/36 | <0,1^{c} | | |
| HWGNHSKSHPQR | 6/36 | <0,1 | | |
| WHKAVPRWLASP | 4/36 | 0,8 | 0,4^{d} | <0,1^{c} |
| HMAHRWQSFLRP | 2/36 | <0,1 | | |
| RVHKRHRTQKNA | 1/36 | >2,5 | <0,1 | |
| HWWKHPTRYSLG | 1/36 | >2,5 | 0,5 | >2,5 |
| HFFKLSNWRTTP | 1/36 | 22,5 | <0,1 | |
| HPLKQYWWRPSI | 1/36 | <0,1 | | 0,4 |

L'immunoréactivité des clones a été déterminée par ELISA comme indiqué dans l'exemple 6, avec 8B4H7^{a} ou 2G2G10^{b} fixé sur la phase solide et les clones de phage sont ajoutés à la dilution finale de 8 x 10¹⁰ phages /ml^{d} et 2 x 10¹⁰ phages /ml^{d}.

### Exemple 7: Localisation des séquences des clones phagiques immunoréactifs sur la séquence de l'HBsAg.

Les séquences en acides aminés des peptides ont été comparées à la séquence de la protéine recombinante d'HBsAg ayant servi à l'obtention de l'anticorps monoclonal (Galibert et al, 1979) en utilisant le logiciel Mac Vector, Ver.4.5 (Kodack). Brièvement, les régions de haute similarité sont détectées avec le programme LFASTA qui recherche les meilleures identités locales (Pearson et Lipman, PNAS 85 : 2444-2448 (1988)). Les deux clones immunoréactifs vis à vis de 2G2G10 ont permis de localiser clairement l'épitope reconnu par 2G2G10 et SB4H7 dans la région 199-208 de HBsAg (Figure 1). En effet, HWWKHPTRYSLG a 4 résidus identiques et 3 résidus similaires avec la séquence 199-208 tandis que HPLKQYWWRPSI partage 3 résidus identiques et 1 résidu similaire avec la séquence 201-205.

### Exemple 8 :Détection de l'antigène HBsAg de type sauvage et de variants

### HBsAg àl'aide des anticorps monoclonaux 2G2G10 et 8B4H7.

Le format utilisé est un format de type sandwich utilisant l'anticorps monoclonal 2G2G10 ou SB4H7 en capture et un anticorps polyclonal de chèvre anti-HBs en détection.

### - Détection de l'antigène HBsAg sauvage dans des sérums de patients :

Des sérums de patients fournis par la Société Nationale de Transfusion Sanguine de Lille comprenant plusieurs sous types (échantillons dilués) ont été testés avec l'anticorps monoclonal 2G2G10 en capture et un anticorps polyclonal de chèvre anti-HBs en détection. Les résultats sont présentés dans le tableau 2 ci-dessous. Les échantillons testés sont identifiés selon leur sous type et leur origine. Les résultats sont exprimés en signal détecté par rapport à une valeur seuil. La valeur seuil correspond à cinq fois le signal de l'échantillon négatif. Les valeurs dont le signal/valeur seuil est supérieur à 1 sont positives.

**Tableau 2**

| Identification de l'échantillon / sous types | Signal / Valeur seuil |
|---|---|
| 41 - adw2 - (US+Asie) | 2,91 |
| 42 - adw4 - (Eur.+Am.sud) | 3,04 |
| 43 - adr - (Asie) | 2,94 |
| 44 - ayw1 | 3,00 |
| 45 - ayw2 - (Europe sud) | 3,94 |
| 46 - ayw3 - (Europe-Asie) | 3,42 |
| 47 - ayw3 | 3,07 |
| 48 - ayw4 - (Afrique) | 4,02 |
| 49 - ayr - (Japon) | 4,00 |
| 50 - Négatif / Diluant des échantillons | 0,2 |

### - Détection de l'antigène HBsAg sauvage dans des plasmas :

Des plasmas de la sérothèque de bioMérieux ont été testés dans les mêmes conditions que ci-dessus en utilisant à la fois l'anticorps monoclonal 2G2G10 et l'anticorps monoclonal SB4H7 en capture. Les résultats sont présentés dans le tableau 3 ci-dessous. Ils sont exprimés en signal/valeur seuil. La valeur seuil correspond à cinq fois le signal de l'échantillon négatif. Les valeurs dont le signal/valeur seuil est supérieur à 1 sont positives.

**Tableau 3**

| Echantillon | 2G2G10 // signal/valeur seuil | 8B4H7 // signal/valeur seuil |
|---|---|---|
| MAP 52 1/600 | 25,7 | 29,4 |
| MAP 97 1/50000 | 24,6 | 36,1 |
| MAP 59 1/3000 | 42,2 | 43,0 |
| MAP 62 1/7000 | 46,0 | 47,6 |
| MAP 64 1/6000 | 54,7 | 70,7 |
| HS0 1/7000 | 58,9 | 56,9 |
| 144519 1/6000 | 52,2 | 54,0 |
| 144344 1/16000 | 62,7 | 69,3 |

### - Détection des variants HBsAg :

L'anticorps 2G2G10 a été testé pour sa capacité à détecter des mutants HBsAg à partir de surnageants de culture des variants HBsAg recombinants qui ont été obtenus auprès du Dr Carman et préparés selon la méthode décrite précédemment par Ireland et al., (Hepatology 31 : 1176-1181(2000)).

Les dosages ont été effectués en utilisant un test qualitatif fluoro-immunoenzymatique développé sur l'analyseur automatique Vidas (marque enregistrée) (Weber et al., Journal of Virological Methods 42 : 63-74 (1993); Mikkelsen et al., Gynecologic Obstetric Investigation 41 : 35-40 (1996)). Ce test de capture en deux étapes a été effectué comme suit :

L'anticorps 2G2G10 été fixé sur le réceptacle phase solide (SPR pour Receptacle Solid Phase) à la concentration finale de 10 µg/ml. Le SPR a ensuite été mis à incuber simultanément avec les surnageants de culture contenant les protéines recombinantes correspondant aux différents variants et l'anticorps polyclonal de chèvre biotinylé anti-HBsAg. La seconde étape a ensuite utilisé le conjugué streptavidine-phosphatase alcaline pour la réaction fluorométrique.

Ainsi que le montre !e tableau 4, en comparaison d'un autre anticorps anti-HBsAg 6H6B6, l'anticorps 2G2G10 est capable de détecter les différents variants testés. En effet, les échantillons BA3.2 et T5N portent respectivement les mutations C124R et T23N qui affectent ainsi que l'ont rapporté Ireland et al., (Hepatology 3 1. : 1176-1181 (2000)) la sécrétion de l'antigène dans les surnageants de cultures testés. L'absence de détection de ces deux surnageants ne peut donc être prise en compte.

**Tableau 4**

| Réactivité des anticorps anti-HBsAg vis à vis de variants HBsAg. | | | |
|---|---|---|---|
| Mutations dans le MHR | Echantillon | 2G2G10 | 6H6B6 |
| Séquence standard | Gly D | 3.3 | 3.0 |
| Séquence standard | Gly Y | 4.9 | 4,3 |
| plasmide contrôle^{a} | | 0.2 | 0.3 |
| P120T | BA 2.4 | 3.3 | 3.5 |
| C121R | J1^{a} | 6.7 | 27.5 |
| T123N | BA 3.4 | 2.2 | 0.3 |
| T123N/C124R | BA 3.2^{a} | 0.2 | 0.3 |
| M133T | SA7 | 3.0 | 2.1 |
| F134L | J2 | 2.3 | 3.0 |
| D144A | AP 3.1 | 4.7 | 0.7 |
| G145R | Arg 1.2 | 7.2 | 0.4 |
| Q129R/G130N/A166V | SA6 | 4.6 | 2.6 |
| Q30R/S53L/L98V/Q101R/S21 0T | HK188 | 27.4 | 24.2 |
| S143L | 1043 Sp | 3.0 | 0.7 |
| P127A/S143L | 1157 Sp | 3.4 | 0.3 |
| P120S/S143L | 1056 Sp | 1.8 | 0.5 |
| Y100S/T118V/R122K/M133I/ Y134N/P142S/S143L/G145K | M5 | 2.0 | 0.7 |
| polysubstitution including D144E | PA17 | 2.5 | 2.3 |
| D99N/122NT123/G145R | T5N^{a} | 0.2 | 0.3 |
| MHR : signifie région hydrophile majeure. | | | |
| Les résultat sont exprimés par le signal moyen de duplicate /une valeur seuil. Pour chaque anticorps, la valeur seuil a été définie par le signal moyen de duplicates obtenus avec 0.16 ng/ml d'HBsAg plasmatique purifié des sous-types , *ad lay.* | | | |

| | | | |
|---|---|---|---|
| ^{a} Echantillons non dilués. | | | |

## Revendications

1. Anticorps monoclonal capable de se lier à un antigène HBsAg de type sauvage et à au moins une, de préférence au moins deux et avantageusement plus de deux, formes mutantes de l'antigène HBsAg, ledit anticorps monoclonal se liant à une séquence peptidique consistant en au moins 6 acides aminés contigus dans la région 199-208 de l'antigène HBsAg, et avantageusement se liant à la séquence peptidique constituée par la région 199-208 de l'antigène HBsAg, selon la numérotation définie dans la figure 1.

2. Anticorps monoclonal selon la revendication 1, capable de se lier à au moins une forme mutante de l'antigène HBsAg qui comprend au moins une substitution d'un acide aminé dans le déterminant « a » de l'antigène HBsAg.

3. Anticorps monoclonal selon la revendication 2, capable de se lier à au moins une forme mutante de l'antigène HBsAg qui comprend au moins une substitution d'un acide aminé dans le déterminant « a » de l'antigène HBsAg aux positions 127, 133, 134, 142, 143, 144 ou 145 de l'antigène HBsAg et éventuellement au moins une autre substitution d'un acide aminé à la position 100, 118, 120, 122 de l'antigène HBsAg.

4. Anticorps monoclonal selon la revendication 3, capable de se lier à la forme mutante de l'antigène HBsAg qui comprend au moins une substitution correspondant en un remplacement d'une sérine par une leucine à la position 143 de l'antigène HBsAg.

5. Anticorps monoclonal selon la revendication 3, capable de se lier à la forme mutante de l'antigène HBsAg qui comprend au moins une substitution correspondant en un remplacement d'un acide aspartique par une alanine à la position 144 de l'antigène HBsAg.

6. Anticorps monoclonal selon la revendication 3, capable de se lier à la forme mutante de l'antigène HBsAg qui comprend au moins une substitution correspondant en un remplacement d'une glycine par une arginine à la position 145 de l'antigène HBsAg.

7. Anticorps monoclonal selon la revendication 3, capable de se lier à la forme mutante de l'antigène HBsAg qui comprend au moins une substitution correspondant en un remplacement d'une proline par une alanine à la position 127 de l'antigène HBsAg et au moins une substitution correspondant en un remplacement d'une sérine par une leucine à la position 143 de l'antigène HBsAg.

8. Anticorps monoclonal selon la revendication 3, capable de se lier à la forme mutante de l'antigène HBsAg qui comprend au moins une substitution correspondant en un remplacement d'une proline par une sérine à la position 120 de l'antigène HBsAg et au moins une substitution correspondant en un remplacement d'une sérine par une leucine à la position 143 de l'antigène HBsAg.

9. Anticorps monoclonal selon la revendication 3, capable de se lier à la forme mutante de l'antigène HBsAg qui comprend une substitution correspondant en un remplacement d'une tyrosine par une sérine à la position 100 de l'antigène HBsAg, une substitution correspondant en un remplacement d'une thréonine par une valine à la position 118 de l'antigène HBsAg, une substitution correspondant en un remplacement d'une arginine par une lysine à la position 122 de l'antigène HBsAg, une substitution correspondant en un remplacement d'une méthionine par une isoleucine à la position 133 de l'antigène HBsAg, une substitution correspondant en un remplacement d'une tyrosine par une asparagine à la position 134 de l'antigène HBsAg, une substitution correspondant en un remplacement d'une proline par une sérine à la position 142 de l'antigène HBsAg, une substitution correspondant en un remplacement d'une sérine par une leucine à la position 143 de l'antigène HBsAg, et une substitution correspondant en un remplacement d'une glycine par une lysine à la position 145 de l'antigène HBsAg.

10. Anticorps monoclonal selon la revendication 1, capable de se lier à un antigène HBsAg de type sauvage et à au moins un mutant HBsAg portant un déterminant « a » codé par des séquences présentant des mutations ponctuelles au niveau d'un ou plusieurs codons codant pour les acides aminés 127, 133, 134, 142, 143, 144 et 145 de l'antigène HBsAg, ledit anticorps monoclonal se liant à la région ou épitope 199-208 de l'antigène HBsAg.

11. Anticorps monoclonal selon l'une quelconque des revendications précédentes, **caractérisé en ce qu'**il appartient à la classe des immunoglobulines G.

12. Anticorps monoclonal selon l'une quelconque des revendications précédentes sous une forme humanisée.

13. Fragment ou dérivé d'un anticorps monoclonal selon l'une quelconque des revendications 1 à 12, choisi parmi le groupe comprenant les fragments Fab, Fab', F(ab)2 et scFv.

14. Procédé pour produire un hybridome capable de produire un anticorps monoclonal selon l'une quelconque des revendications 1 à 12 qui consiste à immuniser un animal mammifère non humain, de préférence murin, avec un antigène HBsAg de type sauvage ou une forme mutante d'un antigène HBsAg, éventuellement sous la forme d'un fragment ou d'un dérivé antigénique approprié, à immortaliser les cellules productrices d'anticorps pour former des hybridomes, à cribler les cultures d'hybridomes contre de l'antigène HBsAg de type sauvage et au moins une forme mutante telle que définie dans l'une quelconque des revendications 2 à 10, éventuellement sous la forme d'un fragment ou dérivé antigénique approprié, et à sélectionner les hybridomes qui produisent des anticorps qui se lient à l'antigène HBsAg de type sauvage et à au moins une forme mutante telle que définie dans l'une quelconque des revendications 2 à 10.

15. Hybridome capable de produire un anticorps monoclonal selon l'une quelconque des revendications 1 à 12.

16. Procédé pour produire un anticorps monoclonal selon l'une quelconque des revendications 1 à 12, qui consiste à cultiver un hybridome selon la revendication 15 ou susceptible d'être obtenu selon un procédé tel que défini dans la revendication 14 *in vitro* ou *in vivo* et à récupérer l'anticorps monoclonal à partir du milieu de culture.

17. Procédé de détection d'antigène HBsAg dans un échantillon, qui consiste à mettre en contact l'échantillon avec au moins un anticorps monoclonal tel que défini dans l'une quelconque des revendications 1 à 11, ou un fragment ou dérivé tel que défini dans la revendication 13 et la revendication 12 et à mettre en évidence la présence d'au moins un complexe antigène/anticorps, fragment ou dérivé.

18. Procédé selon la revendication 17, qui consiste à mettre en contact l'échantillon avec un anticorps monoclonal tel que défini dans l'une quelconque des revendications 1 à 11, ou un fragment ou dérivé tel que défini dans la revendication 13 et la revendication 12 et au moins un autre anticorps choisi parmi les anticorps polyclonaux ou monoclonaux anti-HBsAg.

19. Procédé selon la revendication 17 ou 18 réalisé selon la technique dite sandwich ou de compétition.

20. Procédé selon l'une quelconque des revendications 17 à 19, dans lequel la détection de l'antigène HBsAg est effectué simultanément avec la détection d'anticorps anti-HBc.

21. Composition diagnostique qui comprend au moins un anticorps monoclonal tel que défini dans l'une quelconque des revendications 1 à 11, ou un fragment ou dérivé tel que défini dans la revendication 13 et la revendication 12, éventuellement en association avec au moins un réactif pour la détection d'anticorps anti-HBc.

22. Composition diagnostique selon la revendication 21, qui comprend de plus au moins un autre anticorps monoclonal ou polyclonal pour la détection de l'antigène HBsAg.

23. Antisérum qui comprend un anticorps monoclonal tel que défini dans l'une quelconque des revendications 1 à 11, ou un fragment ou dérivé tel que défini dans la revendication 13 et la revendication 12 ou leurs combinaisons.

24. Antisérum selon la revendication 23, qui comprend également au moins anticorps monoclonal ou un anticorps polyclonal ou un mélange d'anticorps monoclonaux et polyclonaux anti-HBsAg, autres que l'anticorps monoclonal défini dans l'une quelconque des revendications 1 à 11 ou d'un fragment ou dérivé tel que défini dans la revendication 13 et la revendication 12.

25. Composition pharmaceutique appropriée pour une utilisation thérapeutique ou prophylactique pour une immunisation passive contre HBV qui comprend un anticorps monoclonal tel que défini dans l'une quelconque des revendications 1 à 11, ou un fragment ou dérivé tel que défini dans la revendication 13 et la revendication 12 ou leurs combinaisons, en mélange avec un support pharmaceutiquement acceptable.

26. Composition pharmaceutique appropriée pour une utilisation thérapeutique ou prophylactique pour une immunisation passive contre HBV selon la revendication 25, qui comprend également au moins anticorps monoclonal ou un anticorps polyclonal ou un mélange d'anticorps monoclonaux et polyclonaux anti-HBsAg, autres que l'anticorps monoclonal défini dans l'une quelconque des revendications 1 à 11 ou d'un fragment ou dérivé tel que défini dans la revendication 13 et la revendication 12.

27. Anticorps anti-idiotype à un anticorps monoclonal tel que défini dans l'une quelconque des revendications 1 à 11 ou d'un fragment ou dérivé tel que défini dans la revendication 13 et la revendication 12.

28. Composition vaccinale qui comprend une quantité suffisante, pour immuniser un animal contre HBV, d'une composition active biologiquement comprenant un anticorps monoclonal anti-idiotype selon la revendication 27 qui induit chez l'animal la production d'un anticorps anti-anti-idiotype qui reconnaît la région 199-208 de l'antigène HBsAg ou un fragment Fab dudit anticorps anti-idiotype ou leur mélange et un adjuvant pharmaceutiquement acceptable.

29. Peptide immunogène qui consiste en la séquence peptidique commençant à l'acide aminé 199 et se terminant à l'acide aminé 208 de l'antigène HBsAg et est représentée en SEQ ID NO : 1.

30. Peptide immunogène dont la séquence peptidique consiste en la séquence SEQ ID NO : 2.

31. Peptide immunogène dont la séquence peptidique consiste en la séquence SEQ ID NO : 3.

32. Procédé d'obtention d'anticorps monoclonaux selon la revendication 16, de fragments d'anticorps monoclonaux, ou de dérivés d'anticorps monoclonaux en utilisant un peptide immunogène selon l'une quelconque des revendications 29 à 31.

33. Sonde nucléique ou amorce capable de s'hybrider, dans des conditions stringentes, à une séquence nucléotidique ADN qui code pour la séquence peptidique de la revendication 29 ou à la séquence nucléotidique ADN complémentaire de ladite séquence nucléotidique codant pour la séquence peptidique de la revendication 29 ou au produit de transcription ARN desdites séquences nucléotidiques ADN.

34. Sonde nucléique ou amorce selon la revendication 33 capable de s'hybrider, dans des conditions stringentes, à une séquence nucléotidique ADN représentée en SEQ ID NO: 4 qui code pour la séquence peptidique de la revendication 29 ou à la séquence nucléotidique ADN complémentaire de SEQ ID NO : 4 ou au produit de transcription ARN desdites séquences nucléotidiques ADN.

35. Composition diagnostique comprenant au moins une sonde ou au moins une amorce selon l'une des revendications 33 ou 34 pour la détection et/ou la quantification de virus HBV dans un échantillon biologique.

36. Procédé de diagnostic *in vitro* d'ADN et/ou d'ARN de virus HBV dans un échantillon biologique, tel que sérum, plasma ou échantillon tissulaire prélevé chez un patient suspecté d'être infecté par au moins un virus HBV, selon lequel on traite si nécessaire ledit échantillon pour en extraire l'ADN et/ou l'ARN, on met en contact ledit échantillon avec au moins une sonde ou au moins une amorce selon la revendication 33, dans des conditions de stringence détenninées et on détecte et/ou quantifie l'ADN et/ou ARN viral dans l'échantillon, soit par mise en évidence d'une hybridation dudit ADN et/ou ARN viral avec au moins une sonde, soit par amplification dudit ADN et/ou ARN.

## Patentansprüche

1. Monoklonaler Antikörper, der zur Bindung an ein HBsAg-Antigen vom Wildtyp und mindestens eine, vorzugsweise mindestens zwei, vorteilhafterweise mehr als zwei mutierte Formen des HBsAg-Antigens befähigt ist, wobei der monoklonale Antikörper an eine Peptidsequenz bindet, die aus mindestens 6 benachbarten Aminosäuren in der Region 199-208 des HBsAg-Antigens besteht, und vorteilhafterweise an die Peptidsequenz bindet, die aus der Region 199-208 des HBsAg-Antigens besteht, gemäß der in der Fig. 1 definierten Nummerierung.

2. Monoklonaler Antikörper nach Anspruch 1, der zur Bindung an mindestens eine mutierte Form des HBsAg-Antigens befähigt ist, die mindestens eine Substitution einer Aminosäure in der a-Determinante des HBsAg-Antigens aufweist.

3. Monoklonaler Antikörper nach Anspruch 2, der zur Bindung an mindestens eine mutierte Form des HBsAg-Antigens befähigt ist, die mindestens eine Substitution einer Aminosäure in der a-Determinante des HBsAg-Antigens an den Positionen 127, 133, 134, 142, 143, 144 oder 145 des HBsAg-Antigens und gegebenenfalls mindestens eine weitere Substitution einer Aminosäure an der Position 100, 118, 120, 122 des HBsAg-Antigens aufweist.

4. Monoklonaler Antikörper nach Anspruch 3, der zur Bindung an die mutierte Form des HBsAg-Antigens befähigt ist, die mindestens eine einem Ersatz eines Serins durch ein Leucin an der Position 143 des HBsAg-Antigens entsprechende Substitution aufweist.

5. Monoklonaler Antikörper nach Anspruch 3, der zur Bindung an die mutierte Form des HBsAg-Antigens befähigt ist, die mindestens eine einem Ersatz einer Asparaginsäure durch ein Alanin an der Position 144 des HBsAg-Antigens entsprechende Substitution aufweist.

6. Monoklonaler Antikörper nach Anspruch 3, der zur Bindung an die mutierte Form des HBsAg-Antigens befähigt ist, die mindestens eine einem Ersatz eines Glycins durch ein Arginin an der Position 145 des HBsAg-Antigens entsprechende Substitution aufweist.

7. Monoklonaler Antikörper nach Anspruch 3, der zur Bindung an die mutierte Form des HBsAg-Antigens befähigt ist, die mindestens eine einem Ersatz eines Prolins durch ein Alanin an der Position 127 des HBsAg-Antigens entsprechende Substitution und mindestens eine einem Ersatz eines Serins durch ein Leucin an der Position 143 des HBsAg-Antigens entsprechende Substitution aufweist.

8. Monoklonaler Antikörper nach Anspruch 3, der zur Bindung an die mutierte Form des HBsAg-Antigens befähigt ist, die mindestens eine einem Ersatz eines Prolins durch ein Serin an der Position 120 des HBsAg-Antigens entsprechende Substitution und mindestens eine einem Ersatz eines Serins durch ein Leucin an der Position 143 des HBsAg-Antigens entsprechende Substitution aufweist.

9. Monoklonaler Antikörper nach Anspruch 3, der zur Bindung an die mutierte Form des HBsAg-Antigens befähigt ist, die eine einem Ersatz eines Tyrosins durch ein Serin an der Position 100 des HBsAg-Antigens entsprechende Substitution, eine einem Ersatz eines Threonins durch ein Valin an der Position 118 des HBsAg-Antigens entsprechende Substitution, eine einem Ersatz eines Arginins durch ein Lysin an der Position 122 des HBsAg-Antigens entsprechende Substitution, eine einem Ersatz eines Methionins durch ein Isoleucin an der Position 133 des HBsAg-Antigens entsprechende Substitution, eine einem Ersatz eines Tyrosins durch ein Asparagin an der Position 134 des HBsAg-Antigens entsprechende Substitution, eine einem Ersatz eines Prolins durch ein Serin an der Position 142 des HBsAg-Antigens entsprechende Substitution, eine einem Ersatz eines Serins durch ein Leucin an der Position 143 des HBsAg-Antigens entsprechende Substitution und eine einem Ersatz eines Glycins durch ein Lysin an der Position 145 des HBsAg-Antigens entsprechende Substitution aufweist.

10. Monoklonaler Antikörper nach Anspruch 1, der befähigt ist zur Bindung an ein HBsAg-Antigen vom Wildtyp und an mindestens ein mutiertes HBsAg, das eine a-Determinante aufweist, die durch Sequenzen codiert wird, die Punktmutationen in einem oder mehreren Codons aufweisen, die für die Ammosäuren 127, 133, 134, 142, 143, 144 und 145 des HBsAg-Antigens codieren, wobei der monoklonale Antikörper an die Region oder das Epitop 199-208 des HBsAg-Antigens bindet.

11. Monoklonaler Antikörper nach einem der vorhergehenden Ansprüche, **dadurch gekennzeichnet, dass** er zur Klasse G der Immunglobuline gehört.

12. Monoklonaler Antikörper nach einem der vorhergehenden Ansprüche in humanisierter Form.

13. Fragment oder Derivat eines monoklonalen Antikörpers nach einem der Ansprüche 1 bis 12, ausgewählt aus der aus den Fragmenten Fab, Fab', F(ab)2 und scFv bestehenden Gruppe.

14. Verfahren zur Herstellung eines Hybridoms, das zur Produktion eines monoklonalen Antikörpers nach einem der Ansprüche 1 bis 12 befähigt ist, umfassend:
Immunisierung eines nicht-menschlichen Säugers, vorzugsweise eines murinen Säugers, mit einem HBsAg-Antigen vom Wildtyp oder mit einer mutierten Form eines HBsAg-Antigens, gegebenenfalls in Form eines Fragmentes oder geeigneten antigenen Derivats, Immortalisierung der Antikörper produzierenden Zellen zur Bildung von Hybridomen, Screening der Hybridom-Kulturen gegen das HBsAg-Antigen vom Wildtyp und mindestens eine mutierte Form, wie sie in einem der Ansprüche 2 bis 10 definiert ist, gegebenenfalls in Form eines Fragments oder geeigneten antigenen Derivats, und Selektion der Hybridome, die Antikörper bilden, die an das HBsAg-Antigen vom Wildtyp und an mindestens eine mutierte Form, wie sie in einem der Ansprüche 2 bis 10 definiert ist, binden.

15. Hybridom, das zur Produktion eines monoklonalen Antikörpers nach einem der Ansprüche 1 bis 12 befähigt ist.

16. Verfahren zur Herstellung eines monoklonalen Antikörpers nach einem der Ansprüche 1 bis 12, umfassend:
Kultivieren eines Hybridoms nach Anspruch 15 oder das nach einem Verfahren erhältlich ist, wie es im Anspruch 14 definiert ist, *in vitro* oder *in vivo,* und Gewinnung des monoklonalen Antikörpers aus dem Kulturmedium.

17. Verfahren zum Nachweis des HBsAg-Antigens in einer Probe, welches Verfahren darin besteht, dass eine Probe mit mindestens einem monoklonalen Antikörper, wie er in einem der Ansprüche 1 bis 11 definiert ist, oder einem Fragment oder Derivat, wie es in Anspruch 13 und Anspruch 12 definiert ist, in Kontakt gebracht und die Anwesenheit von mindestens einem Komplex Antigen / Antikörper, Fragment oder Derivat nachgewiesen wird.

18. Verfahren nach Anspruch 17, welches darin besteht, dass eine Probe mit einem monoklonalen Antikörper, wie er in einem der Ansprüche 1 bis 11 definiert ist, oder einem Fragment oder Derivat, wie es in Anspruch 13 und Anspruch 12 definiert ist, und mindestens einem anderen Antikörper, der aus polyklonalen oder monoklonalen Anti-HBsAg Antikörpern ausgewählt ist, in Kontakt gebracht wird.

19. Verfahren nach Anspruch 17 oder 18, das nach der Sandwich-Methode oder der kompetitiven Methode durchgeführt wird.

20. Verfahren nach einem der Ansprüche 17 bis 19, bei dem der Nachweis des HBsAg-Antigens gleichzeitig mit dem Nachweis von Anti-HBc-Antikörpern erfolgt.

21. Diagnosezusammensetzung, die mindestens einen monoklonalen Antikörper, wie er in einem der Ansprüche 1 bis 11 definiert ist, oder ein Fragment oder Derivat, wie es in Anspruch 13 und Anspruch 12 definiert ist, gegebenenfalls in Kombination mit mindestens einem Reagens zum Nachweis von Anti-HBc-Antikörpern, enthält.

22. Diagnosezusammensetzung nach Anspruch 21, die außerdem mindestens einen anderen monoklonalen oder polyklonalen Antikörper für den Nachweis des HBsAg-Antigens enthält.

23. Antiserum, das einen monoklonalen Antikörper, wie er in einem der Ansprüche 1 bis 11 definiert ist, oder ein Fragment oder Derivat, wie es in Anspruch 13 und Anspruch 12 definiert ist, oder Kombinationen davon enthält.

24. Antiserum nach Anspruch 23, das auch mindestens einen monoklonalen Antikörper oder einen polyklonalen Antikörper oder eine Mischung von monoklonalen und polyklonalen Antikörpern Anti-HBsAg enthält, die andere sind als der in einem der Ansprüche 1 bis 11 definierte monoklonale Antikörper oder ein Fragment oder Derivat, wie es in Anspruch 13 oder Anspruch 12 definiert ist.

25. Pharmazeutische Zusammensetzung, die zur therapeutischen oder prophylaktischen Verwendung zur passiven Immunisierung gegen HBV geeignet ist, welche Zusammensetzung einen monoklonalen Antikörper, wie er in einem der Ansprüche 1 bis 11 definiert ist, oder ein Fragment oder Derivat, wie es in Anspruch 13 und Anspruch 12 definiert ist, oder Kombinationen davon vermischt mit einem pharmazeutisch akzeptablen Träger enthält.

26. Pharmazeutische Zusammensetzung, die zur therapeutischen oder prophylaktischen Verwendung zur passiven Immunisierung gegen HBV geeignet ist, nach Anspruch 25, die auch mindestens einen monoklonalen Antikörper oder einen polyklonalen Antikörper oder eine Mischung von monoklonalen und polyklonalen Antikörpern Anti-HBsAg enthält, die andere sind als der in einem der Ansprüche 1 bis 11 definierte monoklonale Antikörper oder ein Fragment oder Derivat, wie es in Anspruch 13 oder Anspruch 12 definiert ist.

27. Anti-Idiotyp-Antikörper gegen einen monoklonalen Antikörper, wie er in einem der Ansprüche 1 bis 11 definiert ist, oder ein Fragment oder Derivat, wie es in Anspruch 13 und Anspruch 12 definiert ist.

28. Impfzusammensetzung, die eine zum Immunisieren eines Lebewesens gegen HBV ausreichende Menge einer biologisch aktiven Zusammensetzung, die einen monoklonalen Anti-Idiotyp-Antikörper nach Anspruch 27, der bei dem Lebewesen die Bildung eines Anti-Anti-Idiotyp-Antikörpers bewirkt, der die Region 199-208 des HBsAg-Antigens erkennt, oder ein Fab-Fragment des Anti-Idiotyp-Antikörpers oder deren Mischung enthält, und ein pharmazeutisch akzeptables Adjuvans enthält.

29. Immunogenes Peptid, das aus der Peptidsequenz besteht, die mit der Aminosäure 199 des HBsAg-Antigens beginnt und mit der Aminosäure 208 endet und durch SEQ ID NO. 1 dargestellt wird.

30. Immunogenes Peptid, dessen Peptidsequenz aus der Sequenz SEQ ID NO. 2 besteht.

31. Immunogenes Peptid, dessen Peptidsequenz aus der Sequenz SEQ ID NO. 3 besteht.

32. Verfahren zum Erhalten von monoklonalen Antikörpern nach Anspruch 16, Fragmenten monoklonaler Antikörper oder Derivaten monoklonaler Antikörper unter Verwendung eines immunogenen Peptids nach einem der Ansprüche 29 bis 31.

33. Nukleinsonde oder Primer, die/der unter stringenten Bedingungen zur Hybridisierung mit einer DNA-Nukleotidsequenz, die für die Peptidsequenz nach Anspruch 29 codiert, oder mit der komplementären DNA-Nukleotidsequenz der Nukleotidsequenz, die für die Peptidsequenz nach Anspruch 29 codiert, oder mit dem RNA-Transkriptionsprodukt der genannten DNA-Nukleotidsequenzen befähigt ist.

34. Nukleinsonde oder Primer nach Anspruch 33, die/der unter stringenten Bedingungen zur Hybridisierung mit einer durch SEQ ID NO. 4 dargestellten DNA-Nukleotidsequenz, die für die Peptidsequenz nach Anspruch 29 codiert, oder mit der komplementären DNA-Nukleotidsequenz der SEQ ID NO. 4 oder mit dem RNA-Transkriptionsprodukt der genannten DNA-Nukleotidsequenzen befähigt ist.

35. Diagnosezusammensetzung, die mindestens eine Sonde oder mindestens einen Primer nach einem der Ansprüche 33 oder 34 enthält, zum Nachweis und/oder zur quantitativen Bestimmung des HBV-Virus in einer biologischen Probe.

36. Verfahren zur *in-vitro-*Diagnose der DNA und/oder RNA des HBV-Virus in einer biologischen Probe, wie Serum, Plasma oder Gewebeprobe, die einem Patienten entnommen wurde, bei dem eine Infektion mit mindestens einem HBV-Virus vermutet wird, bei welchem Verfahren diese Probe, wenn notwendig, behandelt wird, um die DNA und/oder RNA zu extrahieren, die Probe mit mindestens einer Sonde oder mindestens einem Primer nach Anspruch 33 unter bestimmten stringenten Bedingungen in Kontakt gebracht wird und die virale DNA und/oder RNA in der Probe nachgewiesen und/oder quantitativ bestimmt wird, indem entweder eine Hybridisierung der viralen DNA und/oder RNA mit mindestens einer Sonde nachgewiesen wird oder die DNA und/oder RNA amplifiziert wird.

## Claims

1. A monoclonal antibody capable of binding to a wild-type HBsAg antigen and to at least one, preferably at least two and advantageously more than two, mutant forms of the HBsAg antigen, said monoclonal antibody binding to a peptide sequence consisting of at least 6 contiguous amino acids in the 199-208 region of the HBsAg antigen, and advantageously binding to the peptide sequence constituted by the 199-208 region of the HBsAg antigen, according to the numbering defined in Figure 1.

2. A monoclonal antibody according to claim 1, capable of binding to at least one mutant form of the HBsAg antigen which comprises at least one amino acid substitution in the "a" determinant of the HBsAg antigen.

3. A monoclonal antibody according to claim 2, capable of binding to at least one mutant form of the HBsAg antigen which comprises at least one amino acid substitution in the "a" determinant of the HBsAg antigen at positions 127, 133, 134, 142, 143, 144 or 145 of the HBsAg antigen and optionally at least one other amino acid substitution at position 100, 118, 120, 122 of the HBsAg antigen.

4. A monoclonal antibody according to claim 3, capable of binding to the mutant form of the HBsAg antigen which comprises at least one substitution corresponding to a replacement of a serine by a leucine at position 143 of the HBsAg antigen.

5. A monoclonal antibody according to claim 3, capable of binding to the mutant form of the HBsAg antigen which comprises at least one substitution corresponding to a replacement of an aspartic acid by an alanine at position 144 of the HBsAg antigen.

6. A monoclonal antibody according to claim 3, capable of binding to the mutant form of the HBsAg antigen which comprises at least one substitution corresponding to a replacement of a glycine by an arginine at position 145 of the HBsAg antigen.

7. A monoclonal antibody according to claim 3, capable of binding to the mutant form of the HBsAg antigen which comprises at least one substitution corresponding to a replacement of a proline by an alanine at position 127 of the HBsAg antigen and at least one substitution corresponding to a replacement of a serine by a leucine at position 143 of the HBsAg antigen.

8. A monoclonal antibody according to claim 3, capable of binding to the mutant form of the HBsAg antigen which comprises at least one substitution corresponding to a replacement of a proline by a serine at position 120 of the HBsAg antigen and at least one substitution corresponding to a replacement of a serine by a leucine at position 143 of the HBsAg antigen.

9. A monoclonal antibody according to claim 3, capable of binding to the mutant form of the HBsAg antigen which comprises a substitution corresponding to a replacement of a tyrosine by a serine at position 100 of the HBsAg antigen, a substitution corresponding to a replacement of a threonine by a valine at position 118 of the HBsAg antigen, a substitution corresponding to a replacement of an arginine by a lysine at position 122 of the HBsAg antigen, a substitution corresponding to a replacement of a methionine by an isoleucine at position 133 of the HBsAg antigen, a substitution corresponding to a replacement of a tyrosine by an asparagine at position 134 of the HBsAg antigen, a substitution corresponding to a replacement of a proline by a serine at position 142 of the HBsAg antigen, a substitution corresponding to a replacement of a serine by a leucine at position 143 of the HBsAg antigen, and a substitution corresponding to a replacement of a glycine by a lysine at position 145 of the HBsAg antigen.

10. A monoclonal antibody according to claim 1, capable of binding to a wild-type HBsAg antigen and to at least one mutant HBsAg carrying an "a" determinant coded by sequences having point mutations at the level of one or more codons coding for the amino acids 127, 133, 134, 142, 143, 144 and 145 of the HBsAg antigen, said monoclonal antibodies binding to the 199-208 region or epitope of the HBsAg antigen.

11. A monoclonal antibody according to any one of the preceding claims, **characterized in that** it belongs to the G class of immunoglobulins.

12. A monoclonal antibody according to any one of the preceding claims in a humanized form.

13. A fragment or derivative of a monoclonal antibody according to any one of claims 1 to 12, selected from the group comprising Fab, Fab', F(ab)2 and scFv fragments.

14. A process for producing a hybridoma capable of producing a monoclonal antibody according to any one of claims 1 to 12 which consists of immunizing a non-human mammalian animal, preferably a murine animal, with a wild-type HBsAg antigen or a mutant form of an HBsAg antigen, optionally in the form of a fragment or of an appropriate antigenic derivative, immortalizing the antibody-producing cells in order to form hybridomas, screening the hybridoma cultures against the wild-type HBsAg antigen and at least one mutant form as defined in any one of claims 2 to 10, optionally in the form of a fragment or an appropriate antigen derivative, and selecting the hybridomas which produce antibodies which bind to the wild-type HBsAg antigen and to at least one mutant form as defined in any one of claims 2 to 10.

15. A hybridoma capable of producing a monoclonal antibody according to any one of claims 1 to 12.

16. A process for producing a monoclonal antibody according to any one of claims 1 to 12, which consists of cultivating a hybridoma according to claim 15 or liable to be obtained according to a process as defined in claim 14 *in vitro* or *in vivo* and recovering the monoclonal antibodies from the culture medium.

17. A process for detection of HBsAg antigen in a sample, which consists of bringing the sample into contact with at least one monoclonal antibody as defined in any one of claims 1 to 11, or a fragment or derivative as defined in claim 13 and claim 12 and identifying the presence of at least one antigen/antibody, fragment or derivative complex.

18. A process according to claim 17, which consists of bringing the sample into contact with a monoclonal antibody as defined in any one of claims 1 to 11, or a fragment or derivative as defined in claim 13 and claim 12 and at least one other antibody chosen from the polyclonal or monoclonal anti-HbsAg antibodies.

19. A process according to claim 17 or 18 carried out according to the sandwich or competition technique.

20. A process according to any one of claims 17 to 19, in which the detection of the HBsAg antigen is carried out simultaneously with the detection of anti-HBc antibodies.

21. A diagnostic composition which comprises at least one monoclonal antibody as defined in any one of claims 1 to 11, or a fragment or derivative as defined in claim 13 and claim 12, optionally in combination with at least one reagent for the detection of anti-HBc antibodies.

22. A diagnostic composition according to claim 21, which moreover comprises at least one other monoclonal or polyclonal antibody for the detection of the HBsAg antigen.

23. An antiserum which comprises a monoclonal antibody as defined in any one of claims 1 to 11, or a fragment or derivative as defined in claim 13 and claim 12 or their combinations.

24. An antiserum according to claim 23, which also comprises at least one monoclonal antibody or polyclonal antibody or a mixture of anti-HbsAg monoclonal and polyclonal antibodies, other than the monoclonal antibody defined in any one of claims 1 to 11, or of a fragment or derivative as defined in claim 13 and claim 12.

25. A pharmaceutical composition suitable for a therapeutic or prophylactic use for a passive immunisation against HBV which comprises a monoclonal antibody as defined in any one of claims 1 to 11, or a fragment or derivative as defined in claim 13 and claim 12 or their combinations, in mixture with a pharmaceutically acceptable support.

26. A pharmaceutical composition suitable for a therapeutic or prophylactic use for a passive immunisation against HBV according to claim 25, which also comprises at least one monoclonal antibody or polyclonal antibody or a mixture of anti-HbsAg monoclonal and polyclonal antibodies, other than the monoclonal antibodies defined in any one of claims 1 to 11 or of a fragment or derivative as defined in claim 13 and claim 12.

27. An anti-idiotype antibody to a monoclonal antibody as defined in any one of claims 1 to 11 or a fragment or derivative as defined in claim 13 and claim 12.

28. A vaccinal composition which comprises a quantity sufficient to immunize an animal against HBV, of a biologically active composition comprising an anti-idiotype monoclonal antibody according to claim 27 which induces in the animal the production of an anti-anti-idiotype antibody which recognizes the 199-208 region of the HBsAg antigen or a Fab fragment of said anti-idiotype antibody or their mixture and a pharmaceutically acceptable adjuvant.

29. An immunogenic peptide which consists of the peptide sequence which starts at amino acid 199 and ends at amino acid 208 of the HBsAg antigen and is represented by SEQ ID NO: 1

30. An immunogenic peptide, the peptide sequence of which consists of the sequence SEQ ID NO: 2.

31. An immunogenic peptide, the peptide sequence of which consists of the sequence SEQ ID NO: 3.

32. A process for obtaining monoclonal antibodies according to claim 16, fragments of monoclonal antibodies, or derivatives of monoclonal antibodies by using an immunogenic peptide according to any one of claims 29 to 31.

33. A nucleic probe or primer capable of hybridizing, under stringent conditions, to a DNA nucleotide sequence which codes for the peptide sequence of claim 29 or to the complementary DNA nucleotide sequence of said nucleotide sequence coding for the peptide sequence of claim 29 or to the RNA transcription product of said DNA nucleotide sequences.

34. A nucleic probe or primer according to claim 33 capable of hybridizing, under stringent conditions, to a DNA nucleotide sequence represented in SEQ ID NO: 4 which codes for the peptide sequence of claim 29 or to the complementary DNA nucleotide sequence of SEQ ID NO: 4 or to the RNA transcription product of said nucleotide DNA sequences.

35. A diagnostic composition comprising at least one probe or at least one primer according to one of claims 33 or 34 for the detection and/or quantification of HBV viruses in a biological sample.

36. A process for *in vitro* diagnosis of HBV virus DNA and/or RNA in a biological sample, such as serum, plasma or tissue sample taken from a patient suspected of being infected by at least one HBV virus, according to which, if necessary, said sample is treated in order to extract the DNA and/or the RNA from it, said sample is brought into contact with at least one probe or at least one primer according to claim 33, under specific stringent conditions, and the viral DNA and/or RNA in the sample is detected and/or quantified, either by identifying a hybridization of said viral DNA and/or RNA with at least one probe, or by amplification of said DNA and/or RNA.


### LISTE DE SEQUENCES

<110> BIOMERIEUX
<120> ANTICORPS MONOCLONAUX DIRIGES CONTRE DES VIRUS DE L'HEPATITE B
<130> IFB BIOM TOPE
<140>
   <141>
<160> 4
<170> PatentIn Ver. 2.1
<210> 1
   <211> 10
   <212> PRT
   <213> Séquence artificielle
<220>
   <223> Description de la séquence artificielle: fragment de la protéine HBSAg du virus de l'hépatite B
<400> 1
   Tyr Tyr Trp Gly Pro Ser Leu Tyr Ser Ile
     1               5                  10
<210> 2
   <211> 12
   <212> PRT
   <213> Séquence artificielle
<220>
   <223> Description de la séquence artificielle: fragment de la protéine HBSAg du virus de l'hépatite B
<400> 2
   His Pro Leu Lys Gln Tyr Trp Trp Arg Pro Ser Ile
     1               5                  10
<210> 3 <211> 12
   <212> PRT
   <213> Séquence artificielle
<220>
   <223> Description de la séquence artificielle: fragment de la protéine HBSAg du virus de l'hépatite B
<400> 3
   His Trp Trp Lys His Pro Thr Arg Tyr Ser Leu Gly
     1               5                  10
<210> 4
   <211> 30
   <212> ADN
   <213> Séquence artificielle
<220>
   <223> Description de la séquence artificielle: fragment de la séquence nucléotidique codant pour la protéine HBSAg du virus de l'hépatite B
<400> 4
   tggtattggg ggccaagtct gtacagcatc                    30